# EUROPEAN PATENT APPLICATION

(11) **EP 3 879 117 A1**
(43) Date of publication of application: **15.09.2021**
(21) Application number: 20213519.0
(22) Date of filing: 11.12.2020
(51) Int. Cl.: F04D 25/06, F04D 25/08, F04D 29/60

(54) **AIR PURIFIER**

(30) Priority: 12.12.2019 US 201962947187 P
(71) Applicant: Techtronic Cordless GP, Anderson, SC 29621 (US)
(72) Inventor: VAN BERGEN, Jonathan R., Easley, South Carolina 29642 (US); WHITMIRE, Porter J., Greenville, South Carolina 29609 (US); WILLIAMS, Brianna E., Mauldin, South Carolina 29662 (US); MOODY, Miles Riordan, Greenville, South Carolina 29605 (US); JENKINS, Luke J, Williamston, South Carolina 29697 (US); CLARK, Austin, Seneca, South Carolina 29672 (US); GROVES, Jeffrey, Greenville, South Carolina 29607 (US); KNIGHT, Tyler H., Greenville, South Carolina 29607 (US); HUGHET, Stephen A., Anderson, South Carolina 29621 (US)
(74) Representative: Stevenson-Hill, Jack Patrick

(57) **Abstract**

An air purifier includes a housing having an air inlet and an air outlet, a filter disposed adjacent the air inlet, a blower unit disposed within the housing to draw air through the air inlet and expel air through the air outlet, a battery receptacle configured to engage a battery for providing power to the motor, and a handle coupled to the housing to facilitate transportation of the air purifier. The blower unit includes a motor and an impeller driven by the motor to induce an air flow, and rotation of the impeller induces air flow at a rate between approximately 11.3 cubic meter per minute (400 cubic feet per minute) and approximately 22.7 cubic meter per minute (800 cubic feet per minute).

## Description

### TECHNICAL FIELD

The present disclosure relates to air purifiers, and more particularly to portable air purifiers.

### BACKGROUND

Generally, air purifiers use internal fans to draw air from a surrounding area into a filter to remove contaminants. Clean air is then directed out of the air purifier and back into the surrounding area.

### SUMMARY

In a first aspect, there is provided an air purifier includes a housing having an air inlet and an air outlet; a filter disposed adjacent the air inlet; a blower unit disposed within the housing to draw air through the air inlet and expel air through the air outlet, and a power receptacle configured to selectively receive power from a first type of power source and a second type of power source. The blower unit includes a motor and an impeller driven by the motor to induce an air flow, and rotation of the impeller induces air flow at a rate between approximately 11.3 cubic meter per minute (400 cubic feet per minute) and approximately 22.7 cubic meter per minute (800 cubic feet per minute).

In one embodiment, the first type of power source is a battery pack and the second type of power source is a power cord.

In one embodiment, the air purifier further comprises a switch for toggling between a DC power mode in which the power receptacle is operable to receive electrical current from the first type of power source and an AC power mode in which the power receptacle is operable to receive electrical current from the second type of power source.

In one embodiment, the air purifier further comprises at least one of a converter and an inverter to modify an electrical current supplied from the power source (first type of power source or the second type of power source) to the blower unit.

In one embodiment, the air purifier further comprises a handle coupled to the housing to facilitate transportation of the air purifier.

In one embodiment, the housing includes a mount that is operable to selectively couple the air purifier to a support structure.

In one embodiment, the air outlet includes a mesh screen.

In one embodiment, the air purifier further comprises a control system having a plurality of user input features to control operation of the air purifier.

In one embodiment, the plurality of user input features includes one or more selected from a group consisting of a first power button operable to control the power produced by the blower unit, a second power button operable to turn the blower unit on and off, and a timer button operable to set a timed duration for the blower unit to operate.

In one embodiment, at least one of the air inlet and the air outlet defines a port having an attachment portion configured to receive a hose.

In a second aspect, there is provided an air purifier includes a housing having an air inlet and an air outlet, a filter disposed adjacent the air inlet, a blower unit disposed within the housing to draw air through the air inlet and expel air through the air outlet, a battery receptacle configured to engage a battery for providing power to the motor, and a handle coupled to the housing to facilitate transportation of the air purifier. The blower unit includes a motor and an impeller driven by the motor to induce an air flow, and rotation of the impeller induces air flow at a rate between approximately 11.3 cubic meter per minute (400 cubic feet per minute) and approximately 22.7 cubic meter per minute (800 cubic feet per minute).

In one embodiment, the air purifier further comprises a mount positioned on the housing and operable to couple the air purifier to a support structure.

In one embodiment, the air inlet and the air outlet are on opposite sides of the housing.

In one embodiment, the handle is coupled to a top side of the housing.

In one embodiment, the air purifier further comprises a stand coupled to the housing to position the air purifier above a surface.

In a third aspect, there is provided an air purifier includes a housing, a filter, a blower unit, a battery receptacle supported by the housing, a battery removably coupled to the battery receptacle, and a switch to selectively energize the blower unit. The housing includes an air inlet, an air outlet, and an attachment portion positioned adjacent the air outlet. The air inlet includes a plurality of openings, and the attachment portion is configured to engage a hose. The filter is disposed adjacent the air inlet to filter air passing through the openings of the air inlet. The blower unit is disposed within the housing to draw air through the air inlet and expel air through the air outlet. The blower unit includes a motor and an impeller driven by the motor. The motor and the impeller are positioned downstream relative to the filter, and rotation of the impeller draws air flow through the air inlet and through the filter. The battery receptacle is configured to engage a removable battery pack, and the removable battery pack provides power to the motor to drive rotation of the impeller.

In one embodiment, an air flow generated by the impeller is between approximately 11.3 cubic meter per minute (400 cubic feet per minute) and approximately 22.7 cubic meter per minute (800 cubic feet per minute).

In one embodiment, the blower unit further includes a blower housing, and wherein the motor and the impeller are positioned within the blower housing.

In one embodiment, the air inlet is a first air inlet and the air outlet is a first air outlet, wherein the housing includes a second air inlet and a second air outlet, and wherein the blower unit draws air through the second air inlet and expels air from the second air outlet.

In one embodiment, the blower unit further includes a blower housing, and wherein the second air outlet is adjacent the first air outlet and wherein air drawn into the blower housing is expelled through the second air outlet directly into the first air outlet.

Other aspects of the disclosure will become apparent by consideration of the detailed description and accompanying drawings. Any feature(s) described herein in relation to one aspect or embodiment may be combined with any other feature(s) described herein in relation to any other aspect or embodiment as appropriate and applicable.

### BRIEF DESCRIPTION OF THE DRAWINGS

Embodiments of the invention will now be described, by way of example, with reference to the accompanying drawings in which:
FIG. 1 is a rear perspective view of an air purifier in one embodiment.
FIG. 2 is a front perspective view of the air purifier of FIG. 1.
FIG. 3 is a schematic drawing of the air purifier of FIG. 1.
FIG. 4 is another schematic drawing of the air purifier of FIG. 1.
FIG. 5 is a perspective view of another air purifier according to another embodiment.

### DETAILED DESCRIPTION

Before any embodiments of the disclosure are explained in detail, it is to be understood that the disclosure is not limited in its application to the details of construction and the arrangement of components set forth in the following description or illustrated in the following drawings. The subject matter is capable of other embodiments and of being practiced or of being carried out in various ways. Also, it is to be understood that the phraseology and terminology used herein is for the purpose of description and should not be regarded as limiting. Use of "including" and "comprising" and variations thereof as used herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. In addition, as used herein, the terms "upper," "lower," and other directional terms are not intended to require any particular orientation, but are instead used for purposes of description only.

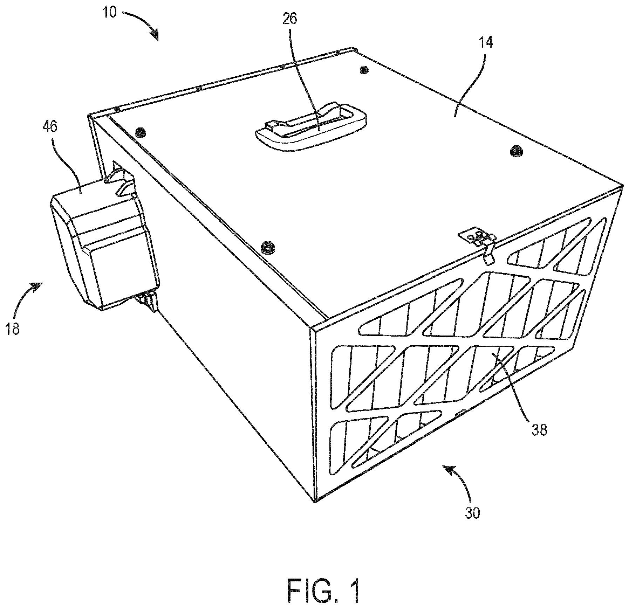

FIGS. 1-4 illustrate an air purifier 10 for use within an interior space, such as a room or enclosed area. The air purifier 10 pulls air from the interior space, removes dust, debris, and other contaminants from the air and blows clean air back into the interior space. The air purifier 10 is operable for both air filtration and dust collection. For dust collection, the air purifier 10 may include an air filter that gathers dust from the air of a wider area or may be dedicated to collect larger dust particles and debris. For air filtration, the air purifier 10 may collect smaller particulates such as odors, pollutants, viruses, bacteria, etc. The air purifier 10 is portable and may be used in a variety of environments. In some embodiments, the air purifier may be positioned on a job site (e.g., in a space being painted), in a crawlspace, in a patient room, etc. In some embodiments, the air purifier 10 may be positioned within the cabin of a work truck, an eighteen-wheeler, or an off-highway truck. In other embodiments, the air purifier 10 may include water filtration capabilities to filter water drawn into the air purifier 10. Further, the air purifier 10 may dehumidify air drawn into the air purifier 10 from the interior space.

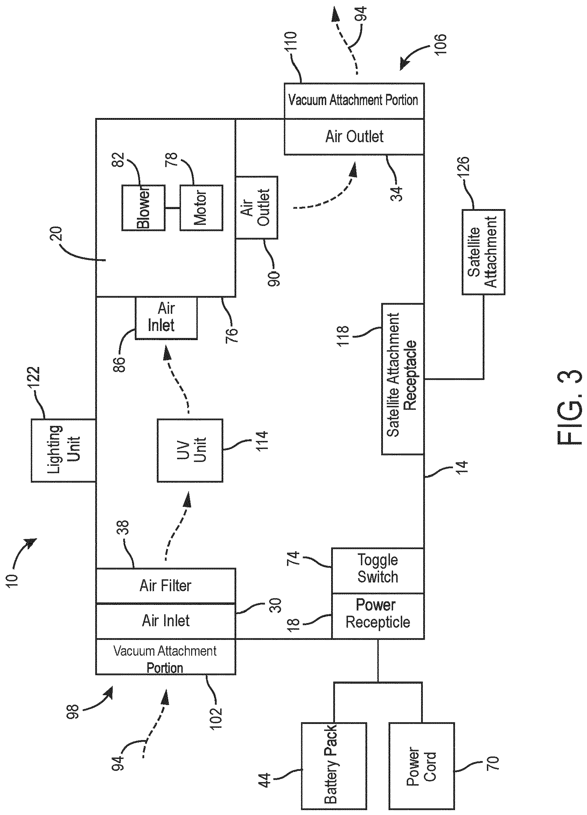

The air purifier 10 includes a housing 14, a power receptacle 18, a blower unit 20 (FIG. 3), and a control system 22. The housing 14 is generally box-shaped and includes a handle 26 on an upper side that a user may grab to carry the air purifier 10 to different rooms or areas. In other embodiments, the housing 14 may be mounted to a ceiling or another structure, as explained in detail below.

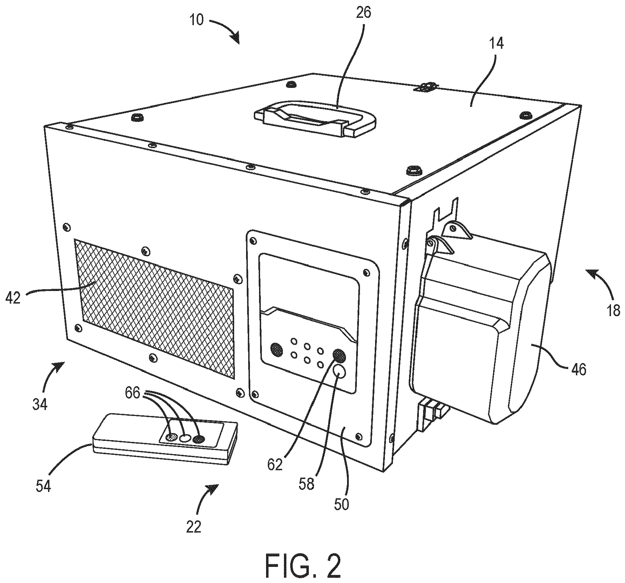

The housing 14 includes an air inlet 30 (FIG. 1) and an air outlet 34 (FIG. 2). In the illustrated embodiment, the air outlet 34 is positioned on an opposing side of the housing 14 from the air inlet 30. The air inlet 30 includes a plurality of apertures that allow air to enter the housing 14 from the surrounding area. A filter 38 is positioned adjacent the air inlet 30, and air passing through the air inlet 30 is drawn through the filter 38. In the illustrated embodiment, the air filter 38 covers the entire air inlet 30. In other embodiments, the air filter 38 may only cover a portion of the air inlet 30. In some embodiments, the filter 38 may be a high efficiency particulate air filter (HEPA filter). In further embodiments, the filter 38 may be positioned within a housing that includes a cyclonic prefilter to improve the life of the filter 38. The filter 38 may also be scented to provide air passing through the filter 38 with a fresh or clean scent. Alternatively, the air purifier 10 may include a reservoir for storing a fluid (e.g., essential oils) to be dispensed into the air. A mesh screen 42 is positioned adjacent the air outlet 34. In some embodiments, the power receptacle 18 is a battery receptacle configured to receive a battery pack 44 (e.g., a DC power source) (FIG. 3) that powers the blower unit 20. The power receptacle 18 includes a cover 46 to protect the battery pack 44 or other power source when a power source is coupled to the power receptacle 18. In the illustrated embodiment, the battery pack 44 is selectively removable from the power receptacle 18.

As shown in FIG. 3, the air purifier 10 may be a hybrid in which the blower unit 20 is capable of being powered by both a DC power source (e.g., the battery pack 44) and an AC power source (e.g., a power cord 70). The power receptacle 18 is electrically coupled to the blower unit 20 to provide power to the blower unit 20. The power receptacle 18 is operable to receive either a portion of the battery pack 44 or a portion of the power cord 70. For example, the power receptacle 18 may include a foot receiver to which the battery pack 44 is coupled, and also may include an outlet to receive a plug or adapter of the power cord 70. In some embodiments, the air purifier 10 may include a special power cord that is adapted to couple to the foot receiver discussed above or another portion of the power receptacle 18. In further embodiments, the AC power source and the DC power source may be received by the power receptacle 18 simultaneously.

Referring to FIG. 3, in some embodiments, the air purifier 10 may include a printed circuit board assembly (PCBA) or controller to detect the type of power being received by the power receptacle 18. The PCBA or controller is operable to determine the type of current being received by the power receptacle 18 without user input and control the current to the blower unit 20. Alternatively, in some embodiments, the air purifier 10 may include a toggle switch 74 positioned adjacent the power receptacle 18 to toggle the type of power source that is providing power through the power receptacle 18. For example, a user may actuate the toggle switch 74 between a first mode (e.g., an AC power mode) in which the power receptacle 18 is operable to receive current from the power cord 70, and a second mode (e.g., a DC power mode) in which the power receptacle 18 is operable to receive current from the battery pack 44. The air purifier 10 may further include an inverter or a converter to change the electrical current received from the power source (e.g., the battery pack 44 or the power cord 70) to match the type of electrical current required for the blower unit 20.

As mentioned, the blower unit 20 is disposed within the housing 14 in a position downstream from the filter 38. The blower unit 20 includes a blower housing 76, a motor 78, and a blower 82 (e.g., a fan blade or an impeller) coupled to the motor 78. The motor 78 and the blower 82 are positioned within the blower housing 76. The blower housing 76 includes an air inlet 86 and an air outlet 90. The air outlet 90 is positioned adjacent the air outlet 34 of the housing 14. Power provided to the power receptacle 18 powers the motor 78 to rotate the blower 82. Rotation of the blower 82 draws an airflow 94 (indicated with arrows) from the area surrounding the air purifier 10 through the air inlet 30 and the filter 38. Dust, debris, and other air contaminants are trapped and removed from the air by the filter 38. Clean air from the airflow 94 is then drawn into the air inlet 86 of the blower housing 76 by the blower 82. The airflow 94 passing through the air purifier 10 may additionally cool the motor 78 and other electronics within the housing 14. The airflow 94 is then expelled from the air outlet 90 of the blower housing 76 and through the air outlet 34 and back into the surrounding areas. In some embodiments, the blower unit 20 is operable to induce an airflow within a range between approximately 8.50 cubic meter per minute (300 cubic feet per minute) and approximately 22.7 cubic meter per minute (800 cubic feet per minute) (CFM). In other embodiments, the blower unit 20 may induce an airflow that is less than 8.50 cubic meter per minute (300 cubic feet per minute) or more than 22.7 cubic meter per minute (800 cubic feet per minute). For example, for applications where the air purifier 10 is to be used for desktop air purification, the blower unit 20 may be operable to induce an airflow within the range of approximately 1.98 cubic meter per minute (70 CFM) and 9.91 cubic meter per minute (350 CFM). Additionally, for applications where the air purifier 10 is to be used for room air purification, the blower unit 20 may be operable to induce an airflow between approximately 11.3 cubic meter per minute (400 CFM) and 17.0 cubic meter per minute (600 CFM). Further, for applications where the air purifier 10 is to be used for room air filtration, the blower unit 20 may be operable to induce an airflow between approximately 8.50 cubic meter per minute (300 cubic feet per minute) and approximately 14.2 cubic meter per minute (500 cubic feet per minute). In addition, in applications where the air purifier 10 is to be used for larger workshop air filtration (e.g., mainly dust particulate), the blower unit 20 may be operable to induce an airflow between approximately 11.3 cubic meter per minute (400 CFM) and 19.8 cubic meter per minute (700 CFM). Furthermore, in applications where the air purifier 10 is dedicated to dust collection within a room, the blower unit 20 may be operable to induce an airflow between approximately 14.2 cubic meter per minute (500 CFM) and 22.7 cubic meter per minute (800 CFM). Moreover, in applications where the air purifier 10 is dedicated to dust collection within a large shop that may include large sanders and planers, the blower unit 20 may be operable to induce an airflow between 22.7 cubic meter per minute (800 CFM) and 28.3 cubic meter per minute (1000 CFM).

In the illustrated embodiment, the air outlet 90 of the blower housing 76 expels the airflow 94 directly into the air outlet 34 of the housing 14. In other embodiments, the air outlet 90 of the blower housing 76 may expel the airflow 94 adjacent the air outlet 34 of the housing 14 to be expelled through the air outlet 34.

In the illustrated embodiment, the air inlet 30 defines an inlet port 98 that is in communication with the surrounding air outside of the air purifier 10. The inlet port 98 includes a first vacuum attachment portion 102 that is may be connected to a vacuum hose. The vacuum hose may be used to draw air through the air inlet 30 containing large particulates. The air outlet 34 defines an exhaust port 106 that is in communication with the surrounding air outside the air purifier 10. The exhaust port 106 defines a second vacuum attachment portion 110 that may be connected to a vacuum hose. The vacuum hose may direct the airflow 94 and particulates within the airflow 94 that is being expelled through the exhaust port 106 into another device to be further purified. Alternatively, the vacuum hose may direct the particulates within the airflow 94 into a storage container to be safely stored. In some embodiments, the air purifier 10 may only include one vacuum attachment portion adjacent either the inlet port 98 or the exhaust port 106.

As shown in FIG. 3, the air purifier 10 also includes an ultraviolet (UV) unit 114. The UV unit 114 includes an ultraviolet light that is capable of further sanitizing and purifying a fluid drawn into the housing 14. The UV unit 114 is disposed within a pathway of the airflow 94. In the illustrated embodiment, the UV unit 114 is positioned downstream of the filter 38. In other embodiments, the UV unit 114 may be positioned upstream of the filter 38 or connected to the filter 38 so that air drawn into the air purifier 10 is filtered and sanitized simultaneously. In further embodiments, the air purifier 10 may include a device that provides electrostatic and ionic filtering to the airflow 94. In other embodiments, the air purifier 10 may include a heating element that heats the airflow 94 as it passes through the air purifier 10, and/or may include a misting device that releases a vapor into the airflow 94.

The air purifier 10 also includes a satellite attachment receptacle 118. The satellite attachment receptacle 118 is supported by the housing 14 and is configured to receive a satellite attachment 126 such as a remote fume extractor or a remote paint booth. The satellite attachment 126 may assist in containing particulate and other particles within the airflow 94. The satellite attachment receptacle 118 may be electrically coupled to the power receptacle 18 to power electronics of the satellite attachment 126.

The air purifier 10 also includes a lighting unit 122 is supported by the housing 14. The lighting unit 122 includes a light for illuminating an area around the air purifier 10. In some embodiments, the light may include light emitting diodes (LEDs).

Operation of the air purifier 10 may be controlled by the control system 22. In the illustrated embodiment, the control system 22 is located adjacent the air outlet 34. As shown in FIG. 2, the control system 22 includes a control board 50. In the illustrated embodiment, a user may interact with the control board 50 via one or more user input features (e.g., buttons 58, 62), and/or may interact by an external device (e.g., a remote control 54, a smart phone, etc.) in communication with the control board 50. A first button 58 may be operated to set the power output of the blower unit 20. For example, the first button 58 may change the blower unit 20 between a low speed, a medium speed, or a high speed. Further, one of the buttons (e.g., the first button 58) may be operated to turn the air purifier 10 on or off. The control board 50 may further include a second button 62 for operating a timer to determine a duration for the blower unit 20 to operate. The control board 50 may be operable to receive signals from the remote control 54 to control the air purifier 10 remotely. As such, the remote control 54 may also include a plurality of user input features 66 to control the operation of the air purifier 10.

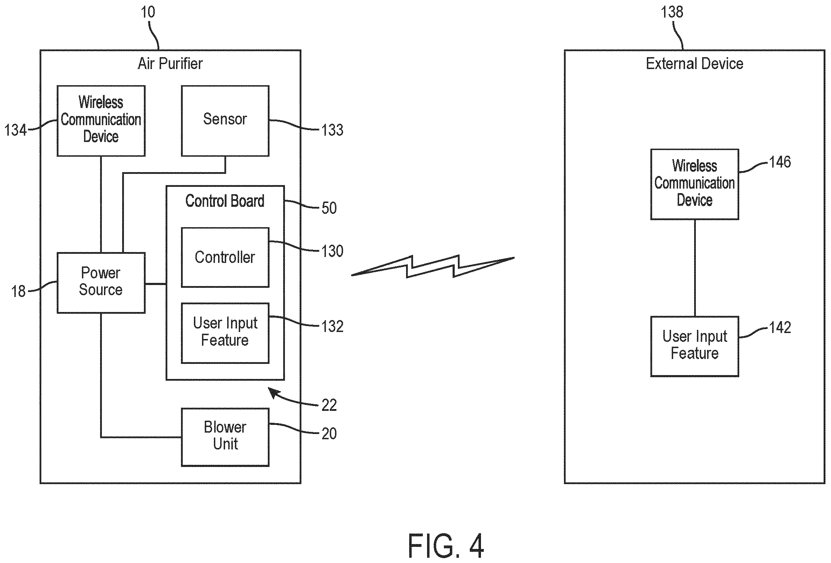

As shown in FIG. 4, in some embodiments the control system 22 further includes a controller 130, a user input feature 132, and a sensor 133. The sensor 133 is operable to send a signal to the controller 130 based on an operating parameter of the air purifier 10. For example, the sensor 133 may be able to sense power provided by the power receptacle 18, or an air quality surrounding the air purifier 10. The blower unit 20, the power receptacle 18, the sensor 133, and the user input feature 132 provide feedback to the controller 130 based on operation of the air purifier 10. The controller 130 can then send a signal indicative of the feedback via a wireless communication device 134 to an external device 138 (e.g. a smart phone) to control operation of the air purifier 10.

For example, the external device 138 may include a user input feature 142 that is operable to send a signal via a wireless communication device 146 to the wireless communication device 134. The signal is processed by the controller 130 to control the power receptacle 18, the blower unit 20, or other features of the air purifier 10. The controller 130 may also be capable of controlling the toggle switch 74 to alternate the type of power received by the blower unit 20. In some embodiments, the controller 130 may default the power receptacle 18 to provide DC power to the blower unit 20 unless a DC power source is not detected. Then, if a DC power source is not detected, the controller 130 may switch the blower unit 20 to receive AC power.

As another example, the controller 130 may be operable to receive a signal from the sensor 133 that an indicator of air quality is below a predetermined threshold. The controller 130 can then automatically turn on the blower unit 20 to purify the air surrounding the air purifier 10 until the air quality is above the predetermined threshold. Alternatively, the controller 130 may run the blower unit 20 for a predetermined amount of time if the air quality is below the predetermined threshold. In some embodiments, the controller 130 may be operable to send a signal via the wireless communication device 134 to the external device 138 indicating that the filter 38 or the battery pack 44 needs to be changed.

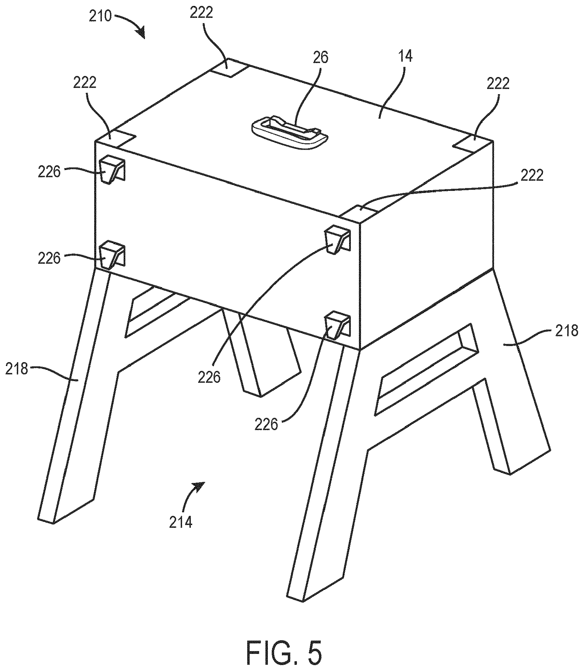

FIG. 5 illustrates an air purifier 210 according to another embodiment. The air purifier 210 is similar to the air purifier 10, and similar features are identified with similar reference numerals. The air purifier 210 includes a stand 214 that is coupled to the housing 14 to support the air purifier 210 above a surface. The stand 214 includes two folding legs 218 that are movable between a tucked or collapsed position, in which the folding legs 218 are adjacent the housing 14 to facilitate transporting the air purifier 10, and a standing position, in which the folding legs 218 are extended to support the air purifier 210 above a surface. In some embodiments, the housing 14 may include a lock, a clip, or a latch to secure the folding legs 218 in the tucked away position.

The air purifier 210 also includes at least one ceiling mount 222 and at least one wall mount 226 to support the air purifier 210 on a ceiling, wall, or other structure. The ceiling mounts 222 may be hooks or brackets that attach to other hooks or brackets supported by a ceiling to support the air purifier 210 on the ceiling. The ceiling mounts 222 are supported on a top side of the housing 14. Similarly, the wall mounts 226 may be hooks or brackets that attach to other hooks or brackets supported by a wall to support the air purifier 210 on the wall. Alternatively, the wall mounts 226 may be recesses or cleats that allow the air purifier 10 to be supported by storage rails. The wall mounts 226 are supported on a lateral side of the housing 14. Both the ceiling mounts 222 and the wall mounts 226 facilitate easy coupling to a wall, ceiling, or other structure so that a user may easily remove and transport the air purifier 10 away from the wall, the ceiling, or other structure. In the illustrated embodiment, the air purifier 210 includes four ceiling mounts 222 and four wall mounts 226. In some embodiments, the air purifier 210 may include more than or less than four wall mounts 226 and ceiling mounts 222.

In some embodiments, the handle 26 may be used to attach the air purifier 210 to a wall or ceiling. For example, a rope may be strung to the handle 26 to hang the air purifier 210 from a structure. In some embodiments, the air purifier 210 may include a strap or harness to allow a user to easily transport the air purifier 210. The strap would allow a user to operate the air purifier 210 as a personal filtration system at any location. In some embodiments, the air purifier 210 may include fold out flanges coupled to the housing 14 that are extendable to fit within an opening. For example, a user may position the air purifier 210 within a window frame and extend the flanges to secure the air purifier 210 with respect to the window frame.

In other embodiments, the air purifier 210 may be attachable to a power tool or other device. For example, the air purifier 210 may be attached to a vacuum to filter particulate that is expelled into the air from the vacuum.

Although aspects have been described in detail with reference to certain embodiments, variations and modifications exist within the scope of one or more independent aspects as described. Various features and advantages are set forth in the claims.

## Claims

1. An air purifier comprising:
a housing having an air inlet and an air outlet;
a filter disposed adjacent the air inlet;
a blower unit disposed within the housing to draw air through the air inlet and expel air through the air outlet, the blower unit including a motor and an impeller driven by the motor to induce an air flow, rotation of the impeller inducing air flow at a rate between approximately 11.3 cubic meter per minute (400 cubic feet per minute) and approximately 22.7 cubic meter per minute (800 cubic feet per minute); and
a power receptacle configured to selectively receive power from a first type of power source and a second type of power source.

2. The air purifier of claim 1, wherein the first type of power source is a battery pack and the second type of power source is a power cord.

3. The air purifier of claim 1 or 2, further comprising a switch for toggling between a DC power mode in which the power receptacle is operable to receive electrical current from the first type of power source and an AC power mode in which the power receptacle is operable to receive electrical current from the second type of power source.

4. The air purifier of claim 3, further comprising at least one of a converter and an inverter to modify an electrical current supplied from the first type of power source or the second type of power source to the blower unit.

5. The air purifier of any one of claims 1 to 4, further comprising a handle coupled to the housing to facilitate transportation of the air purifier.

6. The air purifier of any one of claims 1 to 5,
wherein the housing includes a mount that is operable to selectively couple the air purifier to a support structure; and/or
wherein the air outlet includes a mesh screen; and/or
wherein at least one of the air inlet and the air outlet defines a port having an attachment portion configured to receive a hose.

7. The air purifier of any one of claims 1 to 6, further comprising a control system having a plurality of user input features to control operation of the air purifier.

8. The air purifier of claim 7, wherein the plurality of user input features includes one or more selected from a group consisting of a first power button operable to control the power produced by the blower unit, a second power button operable to turn the blower unit on and off, and a timer button operable to set a timed duration for the blower unit to operate.

9. An air purifier comprising:
a housing having an air inlet and an air outlet;
a filter disposed adjacent the air inlet;
a blower unit disposed within the housing to draw air through the air inlet and expel air through the air outlet, the blower unit including a motor and an impeller driven by the motor to induce an air flow, rotation of the impeller inducing air flow at a rate between approximately 11.3 cubic meter per minute (400 cubic feet per minute) and approximately 22.7 cubic meter per minute (800 cubic feet per minute);
a battery receptacle configured to engage a battery for providing power to the motor; and
a handle coupled to the housing to facilitate transportation of the air purifier.

10. The air purifier of claim 9, further comprising:
a mount positioned on the housing and operable to couple the air purifier to a support structure; and/or
a stand coupled to the housing to position the air purifier above a surface.

11. The air purifier of claim 9 or 10,
wherein the air inlet and the air outlet are on opposite sides of the housing; and/or
wherein the handle is coupled to a top side of the housing.

12. An air purifier comprising:
a housing having an air inlet, an air outlet, and an attachment portion positioned adjacent the air outlet, the air inlet including a plurality of openings, the attachment portion configured to engage a hose;
a filter disposed adjacent the air inlet to filter air passing through the plurality of openings of the air inlet;
a blower unit disposed within the housing to draw air through the air inlet and expel air through the air outlet, the blower unit including a motor and an impeller driven by the motor, the motor and the impeller positioned downstream relative to the filter, rotation of the impeller drawing air flow through the air inlet and through the filter;
a battery receptacle supported by the housing, the battery receptacle configured to engage a removable battery pack, the removable battery pack providing power to the motor to drive rotation of the impeller;
a battery removably coupled to the battery receptacle; and
a switch to selectively energize the blower unit.

13. The air purifier of claim 12,
wherein an air flow generated by the impeller is between approximately 11.3 cubic meter per minute (400 cubic feet per minute) and approximately 22.7 cubic meter per minute (800 cubic feet per minute); and/or
wherein the blower unit further includes a blower housing, and wherein the motor and the impeller are positioned within the blower housing.

14. The air purifier of claim 12 or 13, wherein the air inlet is a first air inlet and the air outlet is a first air outlet, wherein the housing includes a second air inlet and a second air outlet, and wherein the blower unit draws air through the second air inlet and expels air from the second air outlet.

15. The air purifier of claim 14, wherein the second air outlet is adjacent the first air outlet and wherein air drawn into the blower housing is expelled through the second air outlet directly into the first air outlet.
